# EUROPEAN PATENT APPLICATION

(11) **EP 2 584 790 A1**
(43) Date of publication of application: **24.04.2013**
(21) Application number: 11185749.6
(22) Date of filing: 19.10.2011
(51) Int. Cl.: H04Q 9/00, G01N 33/18

(54) **Remote water quality monitoring**

(71) Applicant: Siemens Aktiengesellschaft, 80333 München (DE)
(72) Inventor: Forstmeier, Dieter, 89331 Burgau (DE)

(57) **Abstract**

The present invention relates to a remote water quality monitoring arrangement (1), a distributed water quality monitoring system (20) with said remote water quality monitoring arrangement (1) and method for replacing/ (newly) installing said remote water quality monitoring arrangement (1) in/to said distributed water quality monitoring system (20).

Said remote water quality monitoring arrangement (1) comprises a position detection device (6), for example a GPS-receiver (Global Positioning System), while a current location (7) of said remote water quality monitoring arrangement (1) is automatically detectable/detected.

(Water quality) measurements, i.e. measurement data (4), of said remote water quality monitoring arrangement (1) can automatically be assigned with the detected location (8) facilitating a central server (23) - receiving the measurements, i.e. measurement data (4), together with the detected location (8) of the remote water quality monitoring arrangement (1) - to automatically organize a replacement and/or a new installation of said remote water quality monitoring arrangement (1) in said distributed water quality monitoring system (20).

## Description

### TECHNICAL FIELD OF THE INVENTION AND PRIOR ART

The present invention relates to a remote water quality monitoring arrangement and a distributed water quality monitoring system comprising at least one of said remote water quality monitoring arrangement.

The present invention further relates to method for replacing/installing said remote water quality monitoring arrangement in said distributed water quality monitoring system.

A remote water quality monitoring arrangement and a distributed water quality monitoring system comprising at least one of said remote water quality monitoring arrangement are known from "Wallace & Tiernan®; Hydraclam®, Distribution Water Quality Monitoring, SB.50.700.GE" and "Wallace & Tiernan®; Cloroclam®, Water Quality & Pressure Monitoring".

The remote water quality monitoring arrangement, Chloroclam® Water Quality Monitor, only referred as cloroclam, is an arrangement for online monitoring/measuring of water quality at a remote/distributed measuring (sample) point in/throughout a water distribution system, also referred as a water distribution network, while measuring free or total chlorine residual and pressure (key water quality measurands) at said measuring point.

Made of lightweight corrosion-resistant ABS, chloroclam is readily transportable - operating with battery - and will be fitted underground to a water hydrant installed at said water distribution system/network.

Chloroclam utilises sensors, especially a membrane sensor, for measuring said chlorine residual and pressure of water provided by said water hydrant at said measuring point. While installing chloroclam, respectively chloroclam arrangements, at several measuring points throughout said water distribution system/network water quality measurements can be operated area wide throughout said water distribution system/network - therefore obtaining/controlling the water quality throughout said water distribution system/network.

Said measured data, i.e. said chlorine residual and pressure (water quality data), are recorded and stored internally in chloroclam, respectively in said several chloroclam arrangements, and transmitted from chloroclam, respectively chloroclam arrangements, via GPRS / GSM technology - using an integrated modem - to a central database/server.

Said central database/server can be accessed via a secure web-based interface from any computer connected to the internet for viewing said measured data of chloroclam/the chloroclam arrangements - installed at said measuring points throughout said water distribution system - online.

In addition to viewing the measured data, i.e. water quality data, online, it is possible to export the measured data directly to a PC, especially in a CSV file format, accessible by many of common computer programs (further processing).

The remote water quality monitoring arrangement, Hydraclam®, Distribution Water Quality Monitoring, only referred as hydroclam, is a (nearly) identically water quality monitoring system respectively to chloroclam while hydroclam is measuring/monitoring pressure, temperature, turbidity and conductivity (key water quality measurands) throughout a water distribution system.

Such remote water quality monitoring arrangements, as for example Chloroclam as well as hydroclam, have to be maintained regularly, for example half yearly, in a service hub for replace/renewing a electrical power supply, i.e. a lithium battery, for maintaining membrane probes, recalibration etc.. Since these service hubs can not be done in field, said remote water quality monitoring arrangements - to be maintained - have to be replaced in the field (with new remote water quality monitoring arrangements to be installed).

While said replaced/installed remote water quality monitoring arrangements have other identifications (arrangement ID, i.e. modem ID) these replaced/installed arrangements must also be assigned/identified/addressed (all together referred only as organized), at/by the central server.

Said organizing of said replaced/installed remote water quality monitoring arrangements needs continuous services on/at the central server, as for example the modem/arrangement ID and installation/measuring location/point of said replaced/installed remote water quality monitoring arrangement must be manually entered or updated at the central server.

Mistakes and failures are prepared, especially in wide water distribution systems/networks operating with a high number of said remote water quality monitoring arrangements.

Same situation occurs, if said remote water quality monitoring arrangement will be moved from one measuring point (sample point) to another throughout said water distribution system/network, or if new remote water quality monitoring arrangements will be added/installed to the water distribution system/network at a new measuring point.

For reduction of failures and interchanging data, an additional organization system, e.g. via barcode readers, remote access to the server in the field and/or further databases, can be generated. But from a certain amount of installed arrangements, such a manually updating/coordination of the distributed water quality monitoring system will not be possible.

### SUMMARY OF THE INVENTION

It is a first objective of the invention to provide a remote water quality monitoring arrangement and/or a distributed water quality monitoring system by which the above-mentioned shortcomings can be mitigated.

It is a second objective of the invention to provide a method for replacing and/or installing said remote water quality monitoring arrangement in/to said distributed water quality monitoring system by which the above-mentioned shortcomings can be mitigated.

It is a further objective of the invention to provide a remote water quality monitoring which can operate more reliably and which can be maintained more efficiently, fail-safely and economically.

These objectives are according to the invention achieved by providing a remote water quality monitoring arrangement.

This remote water quality monitoring arrangement comprises a measuring device for measuring a physical condition parameter of water generating measurement data of said measured physical parameter.

Said measured physical condition parameter of said water can be (a) free or total chlorine (residual), pressure, temperature, turbidity, flow rate or conductivity (key water quality measurands) of said water - being used for generating said measurement data accordingly. These key water quality measurands are best in use for identifying/describing water quality as well as controlling a water distribution network/pipe network - and could easily be measured by respective measuring devices/units.

Said measuring device can be a sensor, especially a (online) membrane sensor, further more especially measuring a free and/or total chlorine residual of said water online.

Such a sensor, for example a membrane sensor FC1 (Free Chlorine) or TC1 (Total Chlorine) as well as a bare electrode sensor Depolox5 of Wallace & Tiernan (Wallace & Tiernan, Siemens, Water Technologies, Produktinformation zu Membransensor FC1, TC1 und zu Depolox5 Sensor), is well known, long term stable while measuring and requires less maintenance costs.

The remote water quality monitoring arrangement further comprises a position detection device detecting a (current) global position of said remote water quality monitoring arrangement generating a (current) position information of said remote water quality monitoring arrangement.

Said position detection device can be a GPS receiver detecting said current global position, i.e. a current location, of said remote water quality monitoring arrangement using a GPS system. Said GPS system is commonly used, therefore being highly proved as well as components for using/implementing said GPS system are highly proved and less in costs.

Said position information generated by said position detection device can be geographic coordinates or other mathematical description describing a (geographical) point in a geographical area.

The remote water quality monitoring arrangement also comprises a transmitting unit for transmitting said measurement data together with said position information to a central receiving unit.

Transmitting said measurement data together with said position information will mean that said measurement data are assigned with said position information, especially each measurement data could be assigned with said position information - said measurement data and said position information being transmittable to said central receiving unit.

Said transmitting unit can be a modem transmitting said measurement data together with said position information to said central receiving unit via a GPRS network. Said modem is highly available, as its technology is very common, - and accordingly less in costs.

Said central receiving unit can be a functional component of a central processing unit, for example a central database (system)/server especially being accessible via a secure web-based interface from any computer connected to the internet.

These objectives are according to the invention also achieved by providing a distributed water quality monitoring system.

This distributed water quality monitoring system for monitoring a water quality in a water distribution system comprises at least one of said remote water quality monitoring arrangement arranged at a measuring point in said water distribution system.

Said remote water quality monitoring arrangement can be fitted, where applicable using a mounting adapter, onto a water hydrant of said water distribution system. This low profile installation is less susceptible to tampering and provides increased security as well as said remote water quality monitoring arrangement can easily be removed and/or replaced from fitted hydrants.

This distributed water quality monitoring system further comprises a central processing unit, especially a central database (system)/server.

This central processing unit further comprises said central receiving unit receiving said measurement data together with said position information from said remote water quality monitoring arrangement arranged in said water distribution system.

This central processing unit also comprises a central monitoring unit providing water quality data, especially in kind of a map-view, to a customer. Said water quality data are being generable by using said received measurement data, especially viewing said water quality data online to said customer.

In addition to said viewing the measuring data online, said central processing unit can further be arranged to facilitate an export of said water quality data and/or said measuring data directly to a PC, especially in a CSV file format, accessible by many of common computer programs (further processing) .

While said central processing unit can also be accessible via a secure web-based interface from a computer connected to an internet said water quality data provided by said central processing unit, i.e. said central monitoring unit, can easily and/or anywhere accessed by said customer.

These objectives are according to the invention also achieved by providing a method for replacing and/or installing said remote water quality monitoring arrangement in/to said distributed water quality monitoring system.

During said method, said remote water quality monitoring arrangement is replaced/installed at a measuring point in said water distribution system.

Said remote water quality monitoring arrangement replaced/installed at said measuring point in said water distribution system transmits an identification information identifying said remote water quality monitoring arrangement together with said position information of said remote water quality monitoring arrangement to said central receiving unit of said central processing unit.

Said central processing unit will organize, especially identify, said remote water quality monitoring arrangement as a replaced or installed remote water quality monitoring arrangement of said distributed water quality monitoring system.

In other words, the present invention relates to a remote water quality monitoring arrangement, a distributed water quality monitoring system with said remote water quality monitoring arrangement and a method for replacing/(newly) installing said remote water quality monitoring arrangement in said distributed water quality monitoring system.

Said remote water quality monitoring arrangement comprises a position detection device, for example a GPS-receiver (Global Positioning System), while a current location of said remote water quality monitoring arrangement is automatically detectable/detected.

(Water quality) measurements of said remote water quality monitoring arrangement can automatically be assigned with the detected location facilitating a central server system - receiving the measurements, i.e. measurement data, together with the detected location of the remote water quality monitoring arrangement - to automatically organize a replacement and/or a new installation of said remote water quality monitoring arrangement in said distributed water quality monitoring system.

In a preferred embodiment said transmitting unit is a modem transmitting said measurement data together with said position information to said central receiving unit via a GPRS network.

Current mobile modems can easily be replaced with mobile modems comprising GPS receivers to implement localisation and transmitting functionality.

Said modem can have an identification information, especially a modem ID, identifying said modem and/or said remote water quality monitoring arrangement.

Said transmitting unit, i.e. said modem, could further be arranged to transmit said identification information, i.e. said modem ID, together with said measurement data and said position information to said central receiving unit.

In a further preferred embodiment said transmitting unit is arranged to transmit said measurement data together with a time-stamp of said measurement data to said central receiving unit. Said time-stamp, identifying that time when said measurement data is obtained, can be generated by using said GPS system.

Transmit said measurement data together with said time-stamp of said measurement data will mean that said measurement data are assigned with said time-stamp, especially each measurement data could be assigned with said time-stamp - said measurement data and said time-stamp being transmittable to said central receiving unit.

In another preferred embodiment several of said remote water quality monitoring arrangements are arranged at several of said measuring points in said water distribution system. Said central receiving unit - then - will receive said measurement data together with said position information from each of said remote water quality monitoring arrangements arranged in said water distribution system.

The invention will lead to many advantages while operating said inventive remote water quality monitoring arrangement and/or said inventive distributed water quality monitoring system.

An effort for an additional registration system while organizing said distributed water quality monitoring system, i.e. barcode scanner, remote server setting etc., can be reduced and centralized. With the remote water quality monitoring arrangement identification and the current location of the remote water quality monitoring arrangement, it is easy to address the remote water quality monitoring arrangement and to organize data by/on the central processing/receiving unit Much more process clearness for service and costumer can also be obtained by said organization.

A much easier installation and replacement of the remote water quality monitoring arrangement in the field can be facilitated.

The invention - obtaining/transmitting measuring data assigned with location information automatically - can provide an automatic map design on the server.

The invention can facilitate an easier and cleaner analysis of the network data since measuring data are assigned with location information and transmitted to the central processing unit automatically.

Service assistance for maintenance can be given by said automatically generated current global location information of of the remote water quality monitoring arrangement (approach with GPS data), specially to a field locations.

Interchanging measurement data from the central processing unit can be eliminated.

Therefore, the invention will provide a remote water quality monitoring which can operate more reliably and which can be maintained more efficiently, fail-safely and economically increasing product quality and customer value.

Further advantages as well as advantageous features of the invention appear from the following description.

### BRIEF DESCRIPTION OF THE DRAWINGS

With reference to the appended drawing, below follows a specific description of an embodiment of the invention cited as example.

In the drawing:

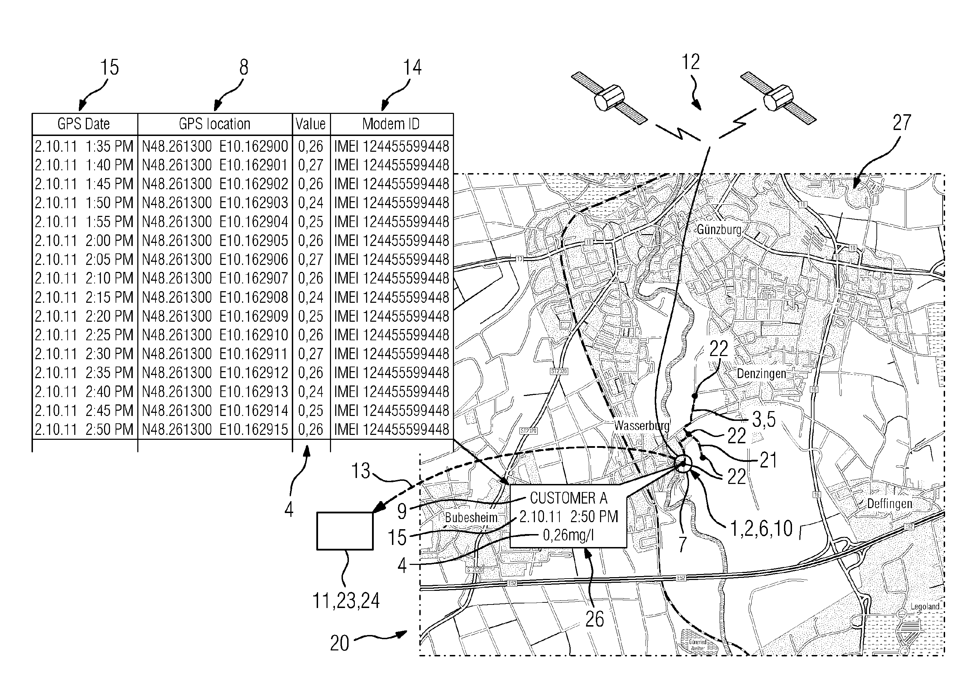

- Figure 1: is a schematic illustration of a distributed water quality monitoring system according to an embodiment of the invention.

### DETAILED DESCRIPTION OF AN EMBODIMENT OF THE INVENTION

### AUTOMATIC LOCATION SYSTEM FOR MONITORING SYSTEMS/ARRANGEMENTS IN WATER DISTRIBUTION NETWORKS/SYSTEMS

The present example are directed to a distributed water quality monitoring system 20, only referred as distributed monitoring system 20, comprising several remote water quality monitoring arrangements 1, only referred as remote monitoring arrangements 1, installed at several measuring points 22 throughout a water distribution system/network 21 controlled by said distributed monitoring system 20.

These remote monitoring arrangements 1 are arrangements for a online monitoring/measuring of water quality at the remote/distributed measuring (sample) points 22 in/throughout the water distribution system 21, while measuring free or total chlorine residual 5 and pressure 5 (key water quality measurands) at a respective measuring point 22.

Made of lightweight corrosion-resistant ABS, these remote monitoring arrangements 1 are readily transportable - operating with battery - and are fitted underground to water hydrants 22, said water hydrants 22 being arranged in said water distribution system/network 21 defining the measuring points 21.

These remote monitoring arrangements 1 utilises sensors 2, especially online membrane sensors 2, for measuring said chlorine residual 5 and pressure 5 of water 3 provided by said water hydrants 22 at said measuring points 22 with generating chlorine measurement data 4 as well as pressure measurement data 4 at each remote monitoring arrangement 1. While installing several of this remote monitoring arrangement 1 at several measuring points 22 throughout said water distribution system/network 21 water quality measurements can be operated area wide throughout said water distribution system/network 21 - therefore obtaining/controlling the water quality throughout said water distribution system/network 21.

Said measured/measurement data 4, i.e. said measured chlorine residual and pressure 4, are recorded and stored internally in the respective remote monitoring arrangement 1.

Each remote monitoring arrangement 1 is supported with a GPRS mobile modem 10 - identifiable with its respective modem ID 14 as well as said respective remote monitoring arrangement 1 will be identifiable by said modem ID - with a GPS-receiver 6 where the current location 7 of the respective remote monitoring arrangement 1 is automatically detected - using the Global Positioning System 12 - while GPS coordinates 8 of the current position 7 of the respective remote monitoring arrangement 1 are generated.

Using a time base of the GPS system 12 each measurement data 4 will be assigned with its respective time-stamp 15 - defining exactly the respective measuring moment- as well as with its respective (measurement) location, i.e. position information 8 of the respective remote monitoring arrangement 1, and respective modem ID 14, identifying the respective remote monitoring arrangement 1.

These data, i.e. measurement data 4, position information 8, time-stamp 15 and modem ID 14, of each remote monitoring arrangement 1 are transmitted - via a GPRS technology, i.e. via a GPRS network 13 - using the GPRS mobile modem 10 - to a central database/server 23 comprising a central receiving unit 11 receiving these data.

The received data can be monitored online by a central monitoring unit 24 of the central database/server 23.

Said central database/server 23 can be accessed via a secure web-based interface from any computer connected to the internet for viewing - edited user-optimized - said received/transmitted data, i.e. water quality data 26, of the remote monitoring arrangements 1 in kind of a map-view 27.

In addition to viewing these data, i.e. water quality data 26, it is possible to export transmitted/received data directly to a PC, especially in a CSV file format, accessible by many of common computer programs.

With the additional GPS location information 8, i.e. the GPS position information 8, it is possible to identify a customer 9 - via the modem ID 14 - and the location 7 (GPS position information) where the water quality measurement was done without interchanging.

Especially if the remote monitoring arrangements 1 are relocated in the water distribution system/network 21 - or new remote monitoring arrangements 1 are relocated in the water distribution system/network 21 - the distributed monitoring system 20 generates important information automatically for organizing the replacement/new installation automatically.

With the modem ID 14 and the position information 8, it is easy to address the remote monitoring arrangement 1 and organize all the transmitted data automatically by the distributed monitoring system 20 on the central server 23.

While each measurement data 4 will be assigned with a location information 8, the replacement and new installation of remote monitoring arrangements 1 in the distribution network 21 can be automatically organized on the central server 23 without additional effords.

### Reference list

- 1: remote (water quality) monitoring arrangement
- 2: measuring device, (online) (membrane) sensor
- 3: water
- 4: measurement data
- 5: physical condition parameter, key water quality measurand, free/total chlorine (residual), pressure, temperature, turbidity, conductivity
- 6: position detection device, GPS receiver
- 7: (current) global position/location
- 8: position information, (GPS) coordinates
- 9: customer
- 10: transmitting unit, (GPRS) modem
- 11: central receiving unit
- 12: GPS system
- 13: GPRS/GSM technology, GPRS/GSM network
- 14: identification information, (modem) ID
- 15: time- stamp

- 20: distributed water quality monitoring system
- 21: water distribution system/network
- 22: measuring point, sample point, water hydrant
- 23: central processing unit, central database/server
- 24: central monitoring unit

- 26: water quality data
- 27: map-view

## Claims

1. A remote water quality monitoring arrangement (1) comprising
- a measuring device (2) for measuring a physical condition parameter (5) of water (3) generating measurement data (4) of said measured physical parameter (5),
- a position detection device (6) detecting a global position (7) of said remote water quality monitoring arrangement (1) generating a position information (8) of said remote water quality monitoring arrangement (1) ,
- a transmitting unit (10) for transmitting said measurement data (4) together with said position information (8) to a central receiving unit (11).

2. A remote water quality monitoring arrangement (1) according to any preceding claim wherein said measured physical condition parameter (5) of said water is free or total chlorine, pressure, temperature, turbidity or conductivity (key water quality measurands) of said water (3).

3. A remote water quality monitoring arrangement (1) according to any preceding claim wherein said measuring device (2) is a membrane sensor, especially measuring a free or total chlorine residual of said water (3), especially measuring said a free or total chlorine residual of said water (3) online.

4. A remote water quality monitoring arrangement (1) according to any preceding claim wherein said position detection device (6) is a GPS receiver (6) detecting a current location (7) of said remote water quality monitoring arrangement (1) using a GPS system (12).

5. A remote water quality monitoring arrangement (1) according to any preceding claim wherein said transmitting unit (10) is a modem (10) transmitting said measurement data (4) together with said position information (8) to said central receiving unit (11) via a GPRS network (13), especially said transmitting unit (10), further more especially said modem (10), having an identification information (14) identifying said transmitting unit (10), further more especially said modem (10), and/or said remote water quality monitoring arrangement (1).

6. A remote water quality monitoring arrangement (1) according to any preceding claim wherein said transmitting unit (10) is arranged to transmit an identification information (14) identifying said remote water quality monitoring arrangement (1) together with said measurement data (4) and said position information (8) to said central receiving unit (11) .

7. A remote water quality monitoring arrangement (1) according to any preceding claim wherein said transmitting unit (10) is arranged to transmit said measurement data (4) together with a time-stamp (15) of said measurement data (4) to said central receiving unit (11), especially said time-stamp (15) generated by using a GPS system (12).

8. A distributed water quality monitoring system (20) for monitoring a water quality in a water distribution system (21) comprising
- at least one of said remote water quality monitoring arrangement (1) according to any preceding claim, arranged at a measuring point (22) in said water distribution system (21), especially fitted onto a water hydrant (22) of said water distribution system (21),
- a central processing unit (23) comprising
- said central receiving unit (11) receiving said measurement data (4) together with said position information (8) from said remote water quality monitoring arrangement (1) arranged in said water distribution system (21) and
- a central monitoring unit (24) providing water quality data (26) to a customer (9), said water quality data (26) being generable by using said received measurement data ( 4 ) .

9. A distributed water quality monitoring system (20) according to claim 8 wherein said central monitoring unit (24) is arranged to provide said water quality data (26) in kind of a map-view (27).

10. A distributed water quality monitoring system (20) according to claim 8 or 9 comprising several of said remote water quality monitoring arrangements (1) according to any preceding arrangement claim, each arranged at one of said measuring point (22) in said water distribution system (21), wherein said central receiving unit (11) is receiving said measurement data (4) together with said position information (8) from each of said remote water quality monitoring arrangements (1) arranged in said water distribution system (21) .

11. A distributed water quality monitoring system (20) according to claim 8, 9 or 10
used for online monitoring of water quality of water (5) of said water distribution system (21) while said central processing unit (23) being accessible via a secure web-based interface from a computer connected to an internet.

12. Method for replacing and/or installing a remote water quality monitoring arrangement (1) in/to a distributed water quality monitoring system (20) according to one of the claims 8 to 11 wherein
- said remote water quality monitoring arrangement (1) is replaced/installed at a measuring point (22) in said water distribution system (21),
- said remote water quality monitoring arrangement (1) replaced/installed at said measuring point (22) in said water distribution system (21) transmits an identification information (14) identifying said remote water quality monitoring arrangement (1) together with said position information (8) of said remote water quality monitoring arrangement (1) to said central receiving unit (11) of said central processing unit (23),
- said central processing unit (23) organizing, especially identifying, said remote water quality monitoring arrangement (1) as a replaced or installed remote water quality monitoring arrangement (1) of said distributed water quality monitoring system (20).
